# EUROPEAN PATENT SPECIFICATION

(11) **EP 2 559 448 B1**
(45) Date of publication and mention of the grant of the patent: **31.05.2017**
(21) Application number: 12177459.0
(22) Date of filing: 10.12.2006
(51) Int. Cl.: A61M 5/00, A61M 5/28, A61M 5/32, A61M 37/00

(54) **Microneedle adapter for dosed drug delivery devices**
Mikronadeladapter für Vorrichtungen zur dosierten Arzneimittelabgabe
Adaptateur de micro-aiguille servant au dosage des dispositifs d'administration de médicament

(30) Priority: 08.12.2005 US 748280 P
(43) Date of publication of application: 20.02.2013
(62) Divisional of application: 06821632.4
(73) Proprietor: Nanopass Technologies Ltd., 74036 Nes Ziona (IL)
(72) Inventor: Levin, Yotam, 74201 Nes Ziona (IL); Yeshurun, Yehushua, 34403 Haifa (IL); Hefetz, Meir, 24954 Israel (IL); Sefi, Yoel, 13845 DN Merom Hagalil (IL); Lavi, Gilad, 74750 Rishon Lezion (IL)
(74) Representative: Modiano, Micaela Nadia

(56) References cited:
- EP-A1- 1 086 718
- WO-A2-02/45771
- US-A- 3 884 229
- US-B1- 6 537 242

## Description

### FIELD AND BACKGROUND OF THE INVENTION

The present invention relates to drug deliver devices and, in particular, it concerns a microneedle adapter for use with dosed drug delivery devices.

Dosed drug delivery devices, often referred to as "pen injectors," are commonly used by diabetics for routine injection of insulin. Similar devices are also used for the delivery of hormones. Dosed drug delivery devices are a preferred means of delivery wherever the volume of drug delivered needs to be variable but accurate, small and frequently administered. Use of the term "pen injector" probably stems from the elongated pen-like form of many of the commercially available devices. However, unless otherwise specified, the term "pen injector" will be used herein interchangeably with the term "dosed drug delivery device" to refer generically to any and all free-standing portable device containing a plurality of doses of a therapeutic liquid which can be operated by a patient for self-injection to deliver metered doses of the liquid to the patient's body on a plurality of occasions. There are various kinds of pen injectors which may be variously classified according to different structural or functional features, such as: devices employing replaceable cartridges and devices which are disposed of when the contents are finished; devices with fixed dosage units or with various dialing and dosing features; devices with different flow activation mechanisms, ergonomics and design, reservoir systems and volume requirements etc.

Pen injectors are used with dedicated replaceable needle assemblies, referred to herein for convenience as "pen needles". Commercially available pen needles known to the inventors all target the subcutaneous (SC) fatty layer and make use of tubular metal components (hypodermic stainless steel needles). Commercially available pen needles typically have lengths ranging from 1mm to 25 mm.

Pen needles are configured to satisfy several requirements unique to pen injectors. On one side, they feature a connector for reversibly connecting to a liquid reservoir within the pen injector. The connector typically includes a hollow needle deployed for piercing a septum (resilient self-sealing membrane) integrated with the liquid cartridge, and an attachment configuration such as a threaded collar for attachment to the pen injector. On the other side, the pen needle features the skin-penetrating needle. The septum-piercing needle and the skin-penetrating needle are typically implemented as opposite ends of a single double-ended needle. The two ends typically have different point shapes, with the rear end configured to avoid coring of a hole in the septum and the front end shaped to minimize pain on penetration through the skin. This renders the double ended needle complex to manufacture. On the other hand, since a single continuous needle is used, there is typically no requirement of sealing between the needle and the surrounding connector body, often allowing the structure to be assembled without the sealing glue required for other hypodermic applications, and the "dead volume" of the needle is very small. For all of the above reasons, design considerations for pen needles are significantly different from those of other hypodermic needles, and such needles have attained a distinct status in the art, often being produced by specialist companies which deal exclusively with pen needles and other pen injector related accessories.

Miniature needles used for pen injectors typically project a minimum of 1 millimeter. In the case of a miniature needle of conventional hypodermic type (i.e., a metal tube formed with a beveled end), the bevel of the needle tip itself typically has a length of at least 0.8 mm, making it impossible to achieve sealed fluid delivery to penetration depths less than 1 mm.

In some published documents, it has been proposed to use "microneedles" as a delivery interface for pen injectors. For the purpose of the present description and claims, the term "microneedle" in its broadest sense is used to refer to a projecting structure with a projecting length of less than 1 millimeter. Examples of such documents include US patent application publication nos. US 2003/0050602 to Pettis and US 2003/0181863 to Ackley et al. WO 02/45771 A2. Theoretically, application of microneedles to pen injectors promises various advantages attributed to intradermal delivery including, but not limited to, altered kinetics (depending on the formulation and the exact injection site, either accelerated absorption, such as may be beneficial for insulin delivery or delayed absorption, for example if a slow release formulation is used), improved response (for example intradermal delivery of vaccines may enhance immune response, allow for smaller doses, potentially lesser booster shots, better vaccination, etc), reduced trauma (since microneedles are smaller than conventional hypodermic needles), and minimally painful or painless injections. The last feature, in particular, is considered highly significant, possibly increasing patient compliance, improving quality of life, improving disease control and reducing expenses on treatment of disease complications. This is particularly relevant in the case of insulin injections for treatment of diabetes due to the direct relation between long term control of blood glucose levels and the prevalence of long term complications.

In practice, implementations of microneedles for pen injectors are not straightforward due to a number of practicat problems. A first major problem of many microneedle designs relates to mechanical weakness of the microneedles which tend to fracture on contact with the skin, particularly when exposed to shear forces due to lateral movement. A further problem is that the highly elastic skin barrier tends to deform around the microneedles without the microneedles penetrating through the stratum corneum (SC). Ah additional problem is that of leakage around the microneedles' point of insertion and/or ejection of the needles by back-pressure generated during injection. Many designs are also prone to blockage of the bores of hollow microneedles due to punching-out of a plug of tissue during insertion through the skin.

Solutions to the aforementioned problems have been suggested in the context of applications such as infusion sets and syringes. Particularly, reference is made to a particularly advantageous robust microneedle structure as taught by US Patent No. 6,533,949, and to various microneedle insertion techniques as taught by PCT Patent Application Publication Nos. WO 03/074102 A2 and WO 2005/049107 A2, and in US Patent Application Publication No. US 2005/0209566 A.

However, these solutions have not previously been adapted to address the particular requirements of pen injectors. Furthermore, given the unique design considerations for pen needles, and the distinct status of pen needles as established in the art, such adaptations are not readily apparent to a person having ordinary skill in the art.

There is therefore a need for an adapter for achieving intradermal dosed delivery of a liquid by use of a dosed drug delivery device.

### SUMMARY OF THE INVENTION

The present invention is an adapter for achieving intradermal dosed delivery of a liquid by use of a dosed drug delivery device.

According to the teachings of the present invention there is provided, an adapter for achieving intradermal dosed delivery of a liquid by use of a dosed drug delivery device, the dosed drug delivery device including a reservoir having a pierceable septum, the adapter comprising: (a) a connector including an attachment configuration for attachment to the dosed drug delivery device and a hollow needle deployed for piercing the septum; (b) a liquid delivery interface mechanically linked to the connector, the liquid delivery interface including a substantially straight skin contact edge and a linear array of hollow microneedles deployed substantially adjacent to, and arrayed substantially parallel to, the skin contact edge, the microneedles projecting away from the skin contact edge; and (c) a flow path arrangement interconnecting the needle and the array of hollow microneedles.

According to a further feature of the present invention, each of the microneedles has a height, and wherein a distance between the skin contact edge and each of the microneedles is no greater than the height of the microneedles.

According to a further feature of the present invention, the substantially straight skin contact edge is formed as an edge of a block of material, the block of material being integrally formed with at least part of the attachment configuration.

According to a further feature of the present invention, an extensional direction of the hollow needle of the connector defines a primary flow axis, and wherein each of the hollow microneedles includes a flow channel defining an injection direction, the injection direction being inclined relative to the primary flow axis by an angle of at least 20 degrees.

According to a further feature of the present invention, the injection direction is inclined relative to the primary flow axis by an angle of between 30 degrees and 150 degrees.

According to a further feature of the present invention, the injection direction is inclined relative to the primary flow axis by an angle of about 90 degrees.

According to a further feature of the present invention, the hollow microneedles are integrally formed with a substrate.

According to a further feature of the present invention, the substrate has a substantially planar surface, and wherein each of the microneedles is formed by at least one wall standing substantially upright from the substantially planar surface and an inclined surface intersecting with the at least one wall.

According to a further feature of the present invention, each of the microneedles has a flow channel passing through the substrate and intersecting with the inclined surface.

According to a further feature of the present invention, the microneedles are formed from silicon.

There is also provided according to the teachings of the present invention, a combination of the aforementioned adapter with a dosed drug delivery device, the combination further including a dosed drug delivery device having a liquid reservoir including a pierceable septum, the adapter being connected to the dosed drug delivery device so that the hollow needle pierces the septum thereby bringing the microneedles into flow connection with contents of the reservoir.

According to a further feature of the present invention, the reservoir contains a quantity of insulin. Alternatively, the reservoir contains a quantity of a fertility hormone. In a further alternative, the reservoir contains a quantity of a growth hormone. In yet a further alternative, the reservoir contains a quantity of a vaccine.

There is also provided according to the teachings of the present invention, an adapter for achieving intradermal dosed delivery of a liquid by use of a dosed drug delivery device, the dosed drug delivery device including a reservoir having a pierceable septum, the adapter comprising: (a) a connector including an attachment configuration for attachment to the dosed drug delivery device and a hollow needle deployed for piercing the septum; (b) a liquid delivery interface mechanically linked to the connector, the liquid delivery interface including a substantially straight skin contact edge and at least one hollow microneedle deployed substantially adjacent to the skin contact edge, the at least one microneedle projecting away from the skin contact edge; and (c) a flow path arrangement interconnecting the needle and the at least one hollow microneedle.

### BRIEF DESCRIPTION OF THE DRAWINGS

The invention is herein described, by way of example only, with reference to the accompanying drawings, wherein:
FIG. 1 is an isometric view of a preferred form of a linear array of microneedles for use in the adapters of the present invention;
FIG. 2A is a exploded isometric view of a first embodiment of an adapter, constructed and operative according to the teachings of the present invention, for use with a dosed drug delivery device to achieve intradermal dosed delivery of a liquid;
FIG. 2B is a isometric partially-cut-away view of the adapter of Figure 2A as assembled prior to use;
FIG. 2C is a cross-sectional view taken through the adapter of Figure 2B after removal of protective covers;
FIG. 3 is a cross-sectional view similar to Figure 2C taken through a second embodiment of an adapter, constructed and operative according to the teachings of the present invention, for use with a dosed drug delivery device to achieve intradermal dosed delivery of a liquid;
FIG. 4A is a side view of the adapter of Figure 2B assembled on a pen injector ready for use;
FIG. 4B is an enlarged view of the region of Figure 4A including the adapter;
FIG. 5A is a side view of the adapter of Figure 3 assembled on a pen injector ready for use;
FIG. 5B is an enlarged view of the region of Figure 5A including the adapter;
FIG. 6A is a view similar to Figure 4B after interfacing of the adapter with the skin of a user;
FIG. 6B is a view similar to Figure 6A after injection of a dose of the liquid;
FIG. 7A is a view similar to Figure 5B after interfacing of the adapter with the skin of a user; and
FIG. 7B is a view similar to Figure 7A after injection of a dose of the liquid.

### DESCRIPTION OF THE PREFERRED EMBODIMENT

The present invention is an adapter for use with a dosed drug delivery device to achieve intradermal dosed delivery of a liquid.

The principles and operation of adapters according to the present invention may be better understood with reference to the drawings and the accompanying description.

By way of introduction, the present invention relates to an adaptation of a microneedle drug delivery interface and corresponding technique described in US Patent Application Publication No. US 2005/0209566 A1 to render it suitable for use as a disposable drug delivery interface for pen injectors. The adapter most preferably employs microneedles produced by MEMS techniques from a single-crystal block of material such as silicon according to the teachings of US Patent No. 6,533,949. Alternatively, various other forms of microneedles and/or other materials may be used, such as are taught in US Patent No. 6,503,231 to Prausnitz et al. These documents are hereby incorporated by reference herein and provide helpful background to the present invention.

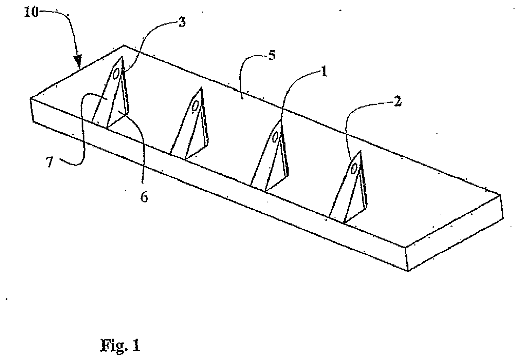

Referring now to the drawings, Figure 1 shows a particularly preferred implementation of a linear array **10** of microneedles for use in the adapter of the present invention. Specifically, linear array **10** includes a number of hollow microneedles, typically between 1 and 10, more preferably between 3 and 6, and in the preferred case illustrated here, 4. Each microneedles has a penetrating point **1,** a liquid flow channel **2** and preferably also a cutting edge **3.** The microneedles are preferably integrally formed with a substrate **5,** having a substantially planar surface. In the preferred implementation shown here, each microneedle is formed by a set of one or more walls **6** standing substantially upright from the substantially planar surface of substrate **5,** and an inclined surface **7** intersecting with walls **6.** Flow channel **2** is preferably formed as a bore passing through substrate 5 and intersecting with inclined surface **7.** The linear microneedle array **10** is preferably formed using a combination of dry etching and wet etching processes from a single crystal of material, most preferably silicon, by techniques such as those described in detail in the aforementioned US Patent No. 6,533,949. Preferred dimensions for the microneedles for this application are a total height in the range of 250 to 650 microns, and most preferably 450 ±30 micron. The flow channel **2** may be round or of other cross-sectional shape, and preferably has a minimum internal diameter of about 45 ±10 microns if round, and an equivalent minimum cross-sectional area if otherwise shaped.

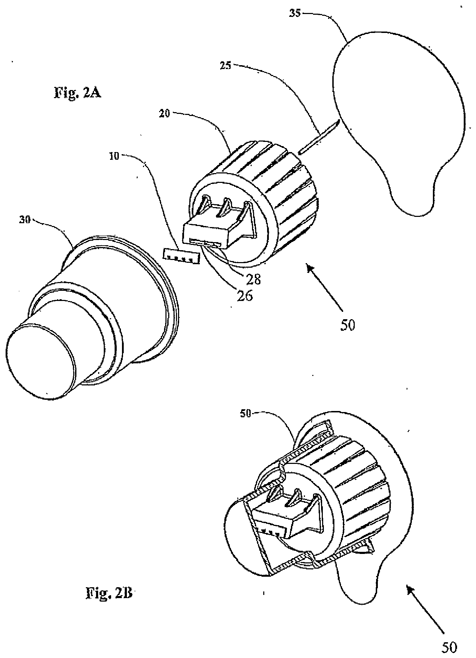

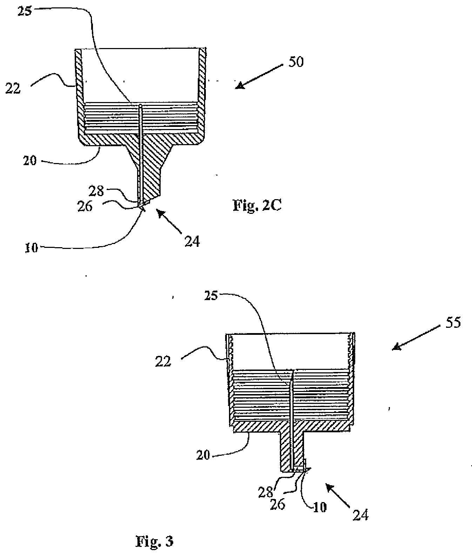

Turning now to Figures 2A-2C, these illustrate a first preferred embodiment of an adapter, generally designated **50,** constructed and operative according to the teachings of the present invention, for achieving intradermal dosed delivery of a liquid by use of a dosed drug delivery device. Generally speaking, adapter **50** includes a connector including an attachment configuration **22** for attachment to a dosed drug delivery device, and a hollow needle **25** deployed for piercing a septum of a reservoir (typically, a cartridge such as a shell vial with moveable plug) of the dosed drug delivery device. Adapter **50** also features a liquid delivery interface **24,** mechanically linked to the connector, including a substantially straight skin contact edge **26** and linear array **10** of hollow microneedles deployed substantially adjacent to, and arrayed substantially parallel to, skin contact edge **26.** The microneedles preferably projecting away from the skin contact edge **26.** A flow path arrangement 28 interconnects needle **25** with the flow channels of the microneedles in linear array **10.**

Prior to use, adapter **50** is preferably protected by a front cover **30,** as shown in Figures 2A and 2B, which protects the microneedles from accidental mechanical damage. Front cover **30** preferably also seals against a peel-off backing sheet **35** which prevents accidental contact with needle **25** and maintains sterility, together forming a pre-sealed sterile packaging for adapter **50.** The device can be sterilized using common methods such as Gamma irradiation or exposure to Ethylene Oxide.

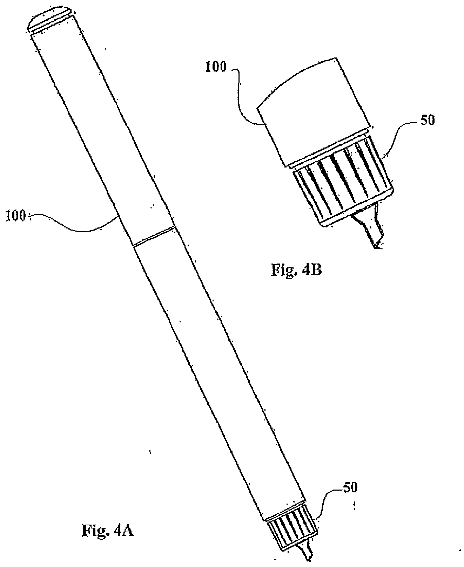

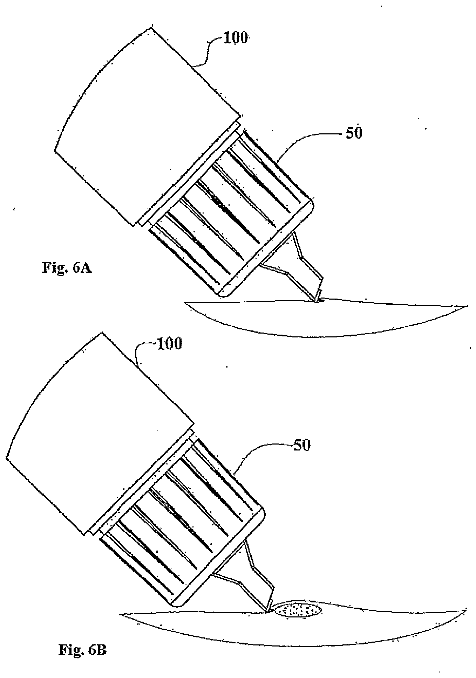

The mode of use of adapter **50** will be understood with reference to Figures 4A, 4B, 6A and 6B. First, after removal of backing sheet **35,** attachment configuration **22** is attached instead of a pen needle to a conventional pen injector **100,** as shown in Figure 4A and enlarged in Figure 4B. As adapter **50** is attached to the pen injector, needle **25** (not visible in this view) pierces the septum of the liquid vial or cartridge within the pen injector, thereby forming a flow connection to the microneedles. Then, as shown in Figure 6A, the assembled device is brought into contact with the user's skin and pushed gently with a vector of motion having a non-zero component parallel to the initial surface of the skin (to the right as shown) so as to achieve penetration with the microneedles projecting primarily sideways, parallel to the initial surface of the skin. This form of insertion achieves numerous advantages over conventional perpendicular insertion, as detailed in the aforementioned US Patent Application Publication No. US 2005/0209566 A1. The pen injector is then actuated in the normal manner to achieve injection of the desired dose of the contained liquid, as illustrated schematically in Figure 6B.

At this stage, it will already be apparent that the adapter of the present invention provides profound advantages over the prior art. Specifically, all pen injector art known to the inventors maintains the conventional approach of perpendicular insertion of the needle(s) into the skin, thereby suffering from the aforementioned limitations of penetration depths in excess of 1 millimeter for conventional needles, or problems of incomplete penetration and ejection by back pressure for microneedles. In contrast, by providing the unique geometry of the present invention in which an array of microneedles are adjacent to a skin contact edge, the present invention facilitates insertion of microneedles so that the microneedle flow channels are directed sideways, i.e., at an angle in the range of ±30° from the initial plane of the skin surface, into tissue not squashed under the device. As a result, the adapter of the present invention allows a pen injector to be used to achieve shallower intradermal liquid delivery than is possible using conventional devices, and is believed to encounter reduced flow impedance and achieve better intradermal distribution than would otherwise be achieved. These and other advantages of the present invention will be better understood with reference to the following description.

Turning now to the features of the present invention in more detail, skin contact edge **26** is preferably formed as an edge of a block of material which supports the microneedle array **10.** Most preferably, this block is integrally formed with at least part of the attachment configuration. Thus, in the example of Figures 2A-2C, adapter **50** is most preferably formed from a combination of only three elements: microneedle array **10,** needle **25,** and a unitary body **20** formed from molded polymer material which provides both the attachment configuration (in this case, a threaded collar) and support for microneedles array 10. Most preferably, body **20** is formed from molded polycarbonate. This three-element implementation minimizes production costs, rendering the adapter suitable for disposable use as a pen needle substitute.

Body **20** also preferably defines any flow paths **28** required to interconnect needle **25** with the flow channels of the microneedles. In the preferred implementation shown, this includes a transverse open channel formed under the point of attachment of microneedles array **10** so that, when the substrate is attached by use of adhesive, welding or other known methods, the channel together with the rear surface of the substrate forms a closed channel for distributing liquid from needle **25** to all of the microneedles. The positioning of this channel is chosen to intersect a central axis of the adapter **50** along which needle **25** is aligned, thereby simplifying manufacture of body **20,** as will be clear to one familiar with plastic injection molding technology.

The form of body **20** is chosen to facilitate bringing the microneedles into contact with the skin in the correct orientation. In the preferred example shown here, body **20** is formed with a forward projecting portion which is roughly rectangular in cross-section, having a major dimension parallel to the extensional direction of microneedle array **10** and a minor dimension perpendicular thereto. The microneedles are preferably deployed with the inclined surface having flow channel 2 facing downwards, i.e., inwards towards the depth of the tissue.

In order to optimize the sideways insertion geometry, the microneedles are preferably close to edge **26.** Preferably, a distance between skin contact edge **26** and each of the microneedles, defined as the distance between edge **26** and the closest part of the base of the microneedles, is no greater than the height of the microneedles themselves as measured perpendicular to the surface of the substrate. Most preferably, the microneedles are juxtaposed with their base starting substantially at edge **26.** Parenthetically, it should be noted that edge **26** itself may be provided by either the edge of the substrate of microneedle array **10** or by an edge of body **20** adjacent to the array **10.**

It will be noted that adapter **50** causes a significant deflection of the flow direction between the axial direction of the dosed drug delivery device (corresponding to the direction of needle **25)** and the injection direction as defined by the flow channels of the microneedles. This deflection is preferably at least about 20 degrees and, more preferably, between about 30 and about 150 degrees. In the case shown here, the deflection is roughly 40 degrees. Nevertheless, in order to achieve an injection direction near parallel to the initial plane of the skin, this embodiment requires deployment of the pen injector at an inclination as shown in Figures 4A, 4B, 6A and 6B.

Parenthetically, although the device is illustrated here in a preferred embodiment in which a linear array of microneedles is used, it should be noted that a minimal embodiment in which a single microneedle is used in proximity to skin contact edge 26 also falls within the broad scope of the present invention.

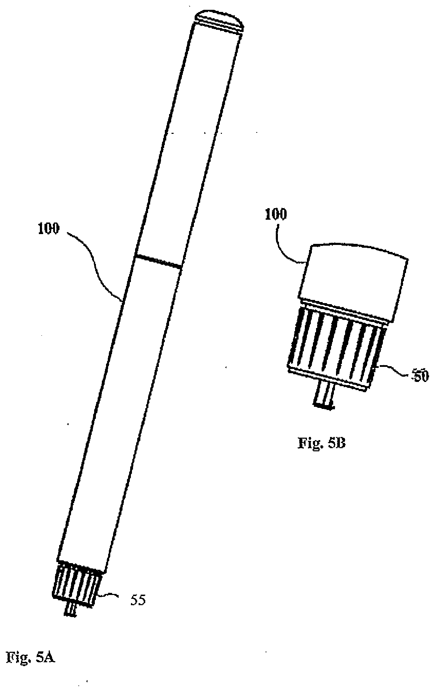

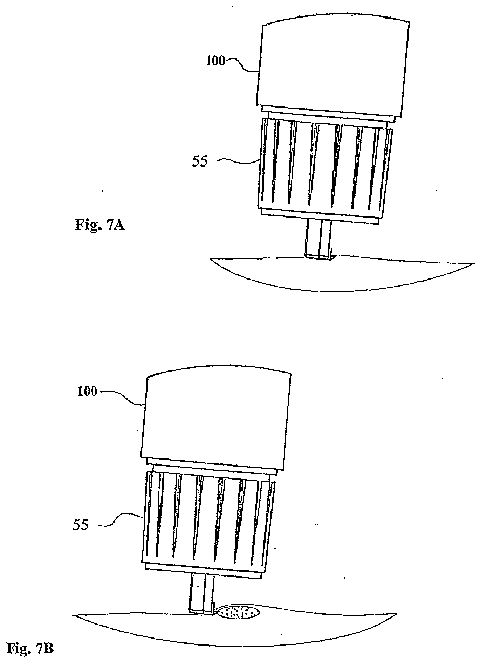

Figures 3, 5A, 5B, 7A and 7B illustrate an alternative embodiment of an adapter, generally designated 55, which provides a larger deflection of the flow direction, namely, about 90 degrees. This orientation achieves sideways injection while allowing the device to be held generally orthogonally to the initial skin surface, in a manner more similar to the orientation of a pen injector used with a conventional pen needle. Initial insertion of the microneedles into the skin surface is preferably performed at a slight angle, as illustrated in Figures 5A and 5B, and typically requires a slight turning motion, applying an anticlockwise turning moment in the orientation as illustrated in Figure 7A.

In other respects, the structural features and function of adapter **55** will be understood by analogy to the corresponding features and function of adapter **50** described above, with like elements being labeled similarly.

It will be appreciated that the present invention may be used to advantage in a large number of drug delivery applications, including both applications for which pen injectors are conventionally used and new applications for which the shallow intradermal delivery achieved by the present invention may be advantageous. Examples of applications include, but are not limited to, administering: insulin, fertility hormones, growth hormone and vaccines. Other applications include, but are not limited to, the substances and modes of treatment mentioned in US Patent Application Publication No. 2005/0163711 A1.

It will be appreciated that the above descriptions are intended only to serve as examples, and that many other embodiments are possible within the scope of the present invention as defined in the appended claims.

## Claims

1. An adapter (50) for use with a dosed drug delivery device having a pierceable septum, the adapter comprising:
(a) a connector including an attachment configuration (22) for attachment to the dosed drug delivery device and a hollow needle (25) deployed for piercing the septum;
(b) a liquid delivery interface (24) mechanically linked to said connector, said liquid delivery interface (24) including a skin contact edge (26) and at least one hollow microneedle deployed adjacent to said skin contact edge (26), said microneedles projecting away from said skin contact edge (26); and
(c) a flow path arrangement interconnecting said needle and said array of hollow microneedles,
**characterised in that** said at least one hollow microneedle is integrally formed with a substrate (5), said microneedle having at least one wall (6) standing upright from a surface, an inclined surface (7) intersecting with said at least one wall (6), and a flow channel (2) passing through said substrate (5) and intersecting with said inclined surface (7), said at least one microneedle being deployed with said inclined surface (7) facing towards said skin contact edge (26).

2. The adapter (50) of claim 1, wherein said at least one microneedle has a height, and wherein a distance between said skin contact edge (26) and said at least one microneedle is no greater than said height of said microneedle.

3. The adapter (50) of claim 1, wherein said skin contact edge (26) is formed as an edge of a block of material, said block of material being integrally formed with at least part of said attachment configuration (22).

4. The adapter (50) of claim 1, wherein an extensional direction of said hollow needle (25) of said connector defines a primary flow axis, and wherein said flow channel (2) defines an injection direction, said injection direction being inclined relative to said primary flow axis by an angle of at least 20 degrees.

5. The adapter (50) of claim 4, wherein said injection direction is inclined relative to said primary flow axis by an angle of between 30 degrees and 150 degrees.

6. The adapter (50) of claim 4, wherein said injection direction is inclined relative to said primary flow axis by an angle of about 90 degrees.

7. The adapter (50) of claim 1, wherein said at least one microneedle is formed from silicon.

8. The adapter (50) of any preceding claim, wherein said at least one hollow microneedle is implemented as a plurality of hollow microneedles deployed in a linear array (10) parallel to said skin contact edge (26).

9. A combination of the adapter (50) of claim 1 with a dosed drug delivery device, the combination further including a dosed drug delivery device having a liquid reservoir including a pierceable septum, the adapter (50) being connected to said dosed drug delivery device so that said hollow needle (25) pierces said septum thereby bringing said microneedles into flow connection with contents of said reservoir.

10. The combination of claim 9, wherein said reservoir contains a quantity of insulin.

11. The combination of claim 9, wherein said reservoir contains a quantity of a fertility hormone.

12. The combination of claim 9, wherein said reservoir contains a quantity of a growth hormone.

13. The combination of claim 9, wherein said reservoir contains a quantity of a vaccine.

## Patentansprüche

1. Adapter (50) zur Verwendung mit einer ein durchstechbares Septum aufweisenden Vorrichtung zur dosierten Arzneimittelabgabe, wobei der Adapter umfasst:
(a) ein Verbindungsstück mit einer Befestigungskonfiguration (22) zur Befestigung an der Vorrichtung zur dosierten Arzneimittelabgabe und einer hohlen Nadel (25), die zum Durchstechen des Septums eingesetzt wird;
(b) eine mechanisch mit dem Verbindungsstück verbundene Flüssigkeitsabgabestelle (24), wobei die Flüssigkeitsabgabestelle (24) eine Hautkontaktkante (26) und mindestens eine hohle Mikronadel umfasst, die neben der Hautkontaktkante (26) eingesetzt ist, wobei die Mikronadeln von der Hautkontaktkante (26) vorragen; und
(c) eine Strömungsweganordnung, die die Nadel und das Array der hohlen Mikronadeln miteinander verbindet, **dadurch gekennzeichnet, dass** mindestens eine hohle Mikronadel einstückig mit einem Substrat (5) gebildet ist, wobei die Mikronadel mindestens eine Wand (6), die senkrecht von einer Oberfläche absteht, eine geneigte Oberfläche (7), die die mindestens eine Wand (6) kreuzt, und einen Strömungskanal (2), der durch das Substrat (5) durchgeht und die geneigte Oberfläche (7) kreuzt, aufweist, wobei die mindestens eine Mikronadel, die mit der geneigten Oberfläche (7) eingesetzt ist, der Hautkontaktkante (26) zugewandt ist.

2. Adapter (50) nach Anspruch 1, wobei die mindestens eine Mikronadel eine Höhe aufweist, und wobei ein Abstand zwischen der Hautkontaktkante (26) und der mindestens einen Mikronadel maximal der Höhe der Mikronadel entspricht.

3. Adapter (50) nach Anspruch 1, wobei die Hautkontaktkante (26) als eine Kante eines Materialblocks gebildet ist, wobei der Materialblock mit mindestens einem Teil der Befestigungskonfiguration (22) einstückig gebildet ist.

4. Adapter (50) nach Anspruch 1, wobei eine Richtung, in die sich die hohle Nadel (25) des Verbindungsstücks erstreckt, eine Hauptströmungsachse definiert, und wobei der Strömungskanal (2) eine Injektionsrichtung definiert, wobei die Injektionsrichtung bezogen auf die Hauptströmungsachse um einen Winkel von mindestens 20 Grad geneigt ist.

5. Adapter (50) nach Anspruch 4, wobei die Injektionsrichtung bezogen auf die Hauptströmungsachse um einen Winkel zwischen 30 Grad und 150 Grad geneigt ist.

6. Adapter (50) nach Anspruch 4, wobei die Injektionsrichtung bezogen auf die Hauptströmungsachse um einen Winkel von ungefähr 90 Grad geneigt ist.

7. Adapter (50) nach Anspruch 1, wobei die min destens eine Mikronadel aus Silizium gebildet ist.

8. Adapter (50) nach einem der vorhergehenden Ansprüche, wobei die mindestens eine hohle Mikronadel als eine Vielzahl hohler Mikronadeln in einem linearen Array (10) parallel zu der Hautkontaktkante (26) implementiert wird.

9. Kombination aus dem Adapter (50) nach Anspruch 1 und einer Vorrichtung zur dosierten Arzneimittelabgabe, wobei die Kombination ferner eine Vorrichtung zur dosierten Arzneimittelabgabe mit einem Flüssigkeitsreservoir aufweist, das ein durchstechbares Septum umfasst, wobei der Adapter (50) derart mit der Vorrichtung zur dosierten Arzneimittelabgabe verbunden ist, dass die hohle Nadel (25) das Septum durchsticht, wodurch die Mikronadeln mit dem Inhalt des Reservoirs in Strömungsverbindung gebracht werden.

10. Kombination nach Anspruch 9, wobei das Reservoir eine Menge an Insulin enthält.

11. Kombination nach Anspruch 9, wobei das Reservoir eine Menge eines Fruchtbarkeitshormons enthält.

12. Kombination nach Anspruch 9, wobei das Reservoir eine Menge eines Wachstumshormons enthält.

13. Kombination nach Anspruch 9, wobei das Reservoir eine Menge eines Impfstoffs enthält.

## Revendications

1. Adaptateur (50) destiné à être utilisé avec un dispositif d'administration de doses de médicament ayant une membrane pouvant être percée, l'adaptateur comprenant :
(a) un raccord comprenant une configuration de fixation (22) pour une fixation au dispositif d'administration de doses de médicament et une aiguille creuse (25) déployée pour percer la membrane ;
(b) une interface (24) de distribution de liquide reliée mécaniquement audit raccord, ladite interface (24) de distribution de liquide comprenant un bord (26) de contact avec la peau et au moins une micro-aiguille creuse déployée de manière adjacente audit bord (26) de contact avec la peau, lesdites micro-aiguilles faisant saillie à l'opposé dudit bord (26) de contact avec la peau ; et
(c) un agencement de trajet d'écoulement interconnectant ladite aiguille et ledit réseau de micro-aiguilles creuses,
**caractérisé par le fait que** ladite au moins une micro-aiguille creuse est formée d'un seul tenant avec un substrat (5), ladite micro-aiguille ayant au moins une paroi (6) s'étendant verticalement à partir d'une surface, une surface inclinée (7) s'intersectant avec ladite au moins une paroi (6), et un canal d'écoulement (2) passant à travers ledit substrat (5) et s'intersectant avec ladite surface inclinée (7), ladite au moins une micro-aiguille étant déployée avec ladite surface inclinée (7) tournée vers ledit bord (26) de contact avec la peau.

2. Adaptateur (50) selon la revendication 1, dans lequel ladite au moins une micro-aiguille a une hauteur, et dans lequel une distance entre ledit bord (26) de contact avec la peau et ladite au moins une micro-aiguille n'est pas supérieure à ladite hauteur de ladite micro-aiguille.

3. Adaptateur (50) selon la revendication 1, dans lequel ledit bord (26) de contact avec la peau est sous la forme d'un bord d'un bloc de matériau, ledit bloc de matériau étant formé d'un seul tenant avec au moins une partie de ladite configuration de fixation (22).

4. Adaptateur (50) selon la revendication 1, dans lequel une direction d'extension de ladite aiguille creuse (25) dudit raccord définit un axe d'écoulement primaire, et dans lequel ledit canal d'écoulement (2) définit une direction d'injection, ladite direction d'injection étant inclinée par rapport audit axe d'écoulement primaire d'un angle d'au moins 20 degrés.

5. Adaptateur (50) selon la revendication 4, dans lequel ladite direction d'injection est inclinée par rapport audit axe d'écoulement primaire d'un angle compris entre 30 degrés et 150 degrés.

6. Adaptateur (50) selon la revendication 4, dans lequel ladite direction d'injection est inclinée par rapport audit axe d'écoulement primaire d'un angle d'environ 90 degrés.

7. Adaptateur (50) selon la revendication 1, dans lequel ladite au moins une micro-aiguille est formée à partir de silicium.

8. Adaptateur (50) selon l'une quelconque des revendications précédentes, dans lequel ladite au moins une micro-aiguille creuse est mise en oeuvre sous la forme d'une pluralité de micro-aiguilles creuses déployées dans un réseau linéaire (10) parallèlement audit bord (26) de contact avec la peau.

9. Combinaison de l'adaptateur (50) de la revendication 1 avec un dispositif d'administration de doses de médicament, la combinaison comprenant en outre un dispositif d'administration de doses de médicament ayant un réservoir de liquide comprenant une membrane pouvant être percée, l'adaptateur (50) étant relié audit dispositif d'administration de doses de médicament de telle sorte que ladite aiguille creuse (25) perce ladite membrane, amenant ainsi lesdites micro-aiguilles en raccordement fluidique avec le contenu dudit réservoir.

10. Combinaison selon la revendication 9, dans laquelle ledit réservoir contient une quantité d'insuline.

11. Combinaison selon la revendication 9, dans laquelle ledit réservoir contient une quantité d'une hormone de fertilité.

12. Combinaison selon la revendication 9, dans laquelle ledit réservoir contient une quantité d'une hormone de croissance.

13. Combinaison selon la revendication 9, dans laquelle ledit réservoir contient une quantité d'un vaccin.
